Europäisches Patentamt

European Patent Office

Office européen des brevets

(11) Publication number: **0 092 334**
**B1**

(12) **EUROPEAN PATENT SPECIFICATION**

(45) Date of publication of patent specification: **01.04.87**

(21) Application number: **83301892.2**

(22) Date of filing: **05.04.83**

(51) Int. Cl.⁴: **A 61 B 17/08**

(54) **Skin closure device.**

(30) Priority: **12.04.82 US 367671**

(43) Date of publication of application:
**26.10.83 Bulletin 83/43**

(45) Publication of the grant of the patent:
**01.04.87 Bulletin 87/14**

(84) Designated Contracting States:
**AT BE CH DE FR GB IT LI LU NL SE**

(56) References cited:
**AU-A- 477 704**
**DE-B-2 038 038**
**US-A-1 452 372**
**US-A-3 516 409**
**US-A-3 825 010**
**US-A-3 863 640**
**US-A-3 933 158**
**US-A-4 073 298**
**US-A-4 114 624**

(73) Proprietor: **Sheehan, Joseph Conrad Mary**
**10s 526 Glenn Drive**
**Burr Ridge Illinois, 60521 (US)**

(72) Inventor: **Sheehan, Joseph Conrad Mary**
**10s 526 Glenn Drive**
**Burr Ridge Illinois, 60521 (US)**

(74) Representative: **Higgins, Michael Roger et al**
**MARKS & CLERK 57/60 Lincoln's Inn Fields**
**London WC2A 3LS (GB)**

Courier Press, Leamington Spa, England.

## Description

The present invention relates to an improved skin closure device in the nature of a surgical appliance which is particularly adapted for use in the closure of skin wounds, whether created surgically or by trauma, which device is adapted to hold the wound in a closed position to facilitate the healing process and to minimize the generation and residue of scar tissue.

It is well recognized that skin wounds, whether created by trauma or as a result of surgery, should be closed as soon as possible to avoid contamination and infection, and to minimize the development of scar tissue.

At the present time, there are three generally recognized methods of skin closure. One involves a simple bandaging of the skin with an adhesive material which involves pulling the skin edges together from edge-to-edge with adhesive straps. Another method involves the suturing of wounds through use of a variety of skin suture methods. The third involves skin closure by stapling the skin edges together. All three techniques have disadvantages hereafter to be described.

In addition to the three basic techniques described above, other more sophisticated devices have been proposed from time to time to facilitate the work of the surgeon in the closure of wounds. One such device is described in U.S. Patent Specification 1 452 372 (Gomez). This prior device comprises two strips of flexible tape which are attached by an adhesive layer one immediately along either side of the wound to be closed. The inner edges of the strips interlock in a zip-like manner to close the tapes over the wound, such interlocking being effected by a special tool provided for that purpose. Furthermore, the rigid interlocking elements at the inner edges of the tapes which make up the zip-like structure carry fixed downwardly extending needles or pins, a plurality of such pins being provided along the length of each tape. The pins automatically penetrate the skin tissue on either side of the wound as the tapes are closed over the wound by the special tool.

This prior device has several disadvantages. First, since the pins are fixed rigidly to the underside of the interlocking closure elements at the inner edges of the tapes they can of necessity give support to the skin tissue only superficially in the epidermal region immediately adjacent the surface of the wound, and cannot in practice be designed to give deep support to the wound. This is because relatively long downwardly extending pins which are rigidly fixed to the closure elements would both interfere with the application of the tapes before the wound was closed, and also be liable to enter the skin at an incorrect angle due to the lack of control which can be exercised when closing this device, it being appreciated that deeply penetrating pins located close to the wound would need to be inserted with a high degree of precision.

Second, again due to their being fixed to the interlocking closure elements, it is not possible to exert individual control over the insertion of the pins into the skin; in other words all the pins are inserted automatically irrespective of whether this would be necessary given the nature of the wound in any particular case, and the surgeon cannot be certain (because he cannot see) whether all the pins have properly penetrated the skin. Also, it is not possible to selectively remove the pins as the wound heals and progressively less pins are required.

Third, since the tape is highly flexible and, furthermore, the wound itself and the area on either side is covered by a dressing, there is little to restrain relative movement between the opposite edges of the wound, so that such edges may become vertically misaligned and/or move apart from one another. Over a period of time such motion would prolong the healing period and lead to the formation of unnecessary scar tissue.

Finally, the zip-like mechanism, like any such mechanism, is liable to jam during closure of the tapes, leading to undesirable trauma to the wound while attempting to free the device or remove and replace it.

To summarise, therefore, this prior device is essentially little different in its effect to a combination of a conventional stitched wound with a tape covering, but is inherently less capable of precise application and may fail during closure. Its only advantage is that of speed of application, but of course this is, or should not except in exceptional cases, be the primary consideration.

For these reasons the device described in U.S. Patent Specification 1 452 372 (Gomez), and like devices which have occasionally been proposed, have never so far as we are aware come into widespread practical use.

A skin closure device according to the precharacterising portion of claim 1 is disclosed in US Patent Specification 3 933 158. However, this device is solely adapted for attachment to the surface of the skin at the wound, and does not provide for deep support of the wound.

It is therefore an object of the present invention to provide a device for the closure of wounds which can effect a rapid closure if necessary but which avoids or mitigates the disadvantages of the known devices.

This object is achieved by the combination of features claimed in claim 1.

The advantages of the invention are:

1) The strips of material which are positioned along either side of the wound are sufficiently rigid to stabilise the device on the skin surface yet sufficiently flexible to permit a degree of twisting and bending of the device to allow the latter to follow the path of the wound where this is not a straight line as will rarely be the case.

2) The pins are slidable relative to the strips so that they can be inserted precisely into the skin, individually or in small groups, in numbers appropriate to the nature of the wound, after the strips have been attached to the surface of the skin. Thus they do not interfere with the initial positioning of the strips. Also, they may be selectively withdrawn fom the wound as the latter heals. Finally, the pins may be designed for driving relatively deeply into the skin to provide support to the dermis and subcutaneous tissue, this again resulting from their being slidable relative to the strips and thus being capable of precise and controlled insertion into the skin after the strips have been applied to the surface of the skin.

3) The adhesive layer may extend to closely adjacent the opposite edges of the wound, and this, together with the securing means which both prevents the abutting inner edges of the strips from mutual separation and limits their relative vertical movement, correspondingly limits such movement over the wound area as a whole by virtue of the semi-rigid construction of the strips.

4) The simple closure means, which essentially comprises a pair of raised portions integral with the inner edges of the strips and held together in abutment, is much simpler in construction than the zip-like mechanism of the prior art and far less likely to fail.

It is to be understood that the pins are not necessarily held in retracted non-penetrating positions in the strips when the latter are applied to the skin. They may in fact be provided as separate items and inserted into pre-tapped locating holes after adherence of the strips to the skin. Furthermore, the adhesive layer may be applied to the skin rather than be provided on the strips; however, the effect is the same once the strips have been attached to the skin.

Embodiments of the invention will now be described, by way of example, with reference to the accompanying drawings, in which:

Figure 1 is a schematic representation of skin tissue defining a wound therein which may be closed by devices of the type disclosed herein.

Figure 2 is a schematic representation of a prior art skin closure device involving the use of an adhesive material.

Figure 3 is a schematic representation of a prior art skin closure means involving the use of a suture.

Figure 3a is a schematic representation of a vertical shift of tissue with a prior art suture closure.

Figure 4 is a schematic representation of a prior art staple closure for a skin wound.

Figure 4a is a schematic representation of a vertical shift of tissue as a result of off-center orientation of the staple of Figure 4 in closure.

Figure 5 is a cross sectional elevation of a first embodiment of a skin closure device according to the present invention.

Figure 5a is a partial side elevation of the device of Figure 5 omitting the closure housing.

Figure 6 is a top plan view of the device of Figure 5 showing the strips and closure housing in partially assembled relation.

Figure 7 is an end view of one of the strips of the device of Figures 5 and 6.

Figure 8 is a segmented side view of one of the strips of the device of Figures 5 and 6.

Figure 9 is an end view of the closure housing of the device of Figures 5 and 6.

Figure 9a is a segmented side elevation of the housing of Figure 9.

Figure 10 is a cross sectional elevational view of the second preferred embodiment of the skin closure device of the present invention.

Figure 11 is a top plan view of a portion of one of the pin harnesses of the embodiment of Figure 10.

Figure 12 is a side elevational view of a portion of the pin harness of Figure 10.

Figure 13 is a sectional view taken along line 13—13 of Figure 11.

Figure 14 is a top plan view of a portion of the housing of the embodiment of Figure 10.

Figure 15 is a side elevational view of a portion of the housing of Figure 14.

Figure 16 is a sectional view taken along line 16—16 of Figure 14.

Figure 17 is a sectional view taken along line 17—17 of Figure 14.

Figure 18 is an enlarged view of an alternative embodiment of a pin suitable for use in the device of Figure 10.

Figure 19 is an enlarged view of a second alternative embodiment of a pin suitable for use in the device of Figure 10.

In general, human skin is made up of many separately defined layers, which for simplicity can be grouped in three commonly defined layers. The outer layer is called the epidermis and includes a layer of dead cells which gradually form a pavement network, layer upon layer. This network forms an impermeable boundary of dead or dying cells at the exterior or superficial layer of the skin. These cells are gradually replaced in a continually evolving process as new layers of cells are produced from within.

The second layer to be discussed here is the germinal epithelium, sometimes called the dermis, which is the true growth area of the skin. The germinal epithelium is a relatively thin layer in relation to the other two layers being generally described here. This basement, or germinal epithelium, is constantly replacing the layer above it and gradually pushing the most superficial layer towards the outside of the skin. The proper joining of this layer is important since this is the layer which, when joined properly end-to-end with a surgical closure device, will give a good or near perfect junction between the dermis on one side of the wound and the dermis of the other side, thereby minimizing the formation of scar tissue. For example, this is the layer which, when burned by deep burns, will often require skin graft. In general, nothing can replace this layer of skin other than germinal epithelium itself, either growing from the side or transplanted when lost.

Beneath the germinal epithelium lies the basement membrane, which is a thin layer of delicate non-cellular material of a fine filamentous texture whose principal component is collagen. This layer gives body and support to the overlying germinal epithelium, and is a fatty, fluid-like layer which provides a generally defined cushioning effect whereby the germinal epithelium can easily slide over the muscles. This subcutaneous tissue has poor adherent qualities and poor healing qualities, of itself, since it is of a semi-fluid character in the normal state and often does not come together well when sutured.

The nature and physical properties of each of the three skin layers generally described above are somewhat different. For example, the outer, crusty layer of stratified epithelium, described as the epidermis, is relatively dehydrated. It generally varies in thickness with the maximum thickness being on the sole of the foot and the minimum thickness being in the facial area. It has the ability to be pulled together and can be joined with an adhesive material to close a wound in the stratified epithelium.

It should be noted, however, that the germinal epithelium and the basement membrane, generally described above, lie at some depth with respect to the epidermis and are critically important to skin closure since they are the primary source of healing with respect to any skin wound.

The basement membrane is made up of high density collagen material which, when cut, has a tendency to recoil and, subsequently, will often result in movement inwards toward the body of the germinal epithelium. The fatty layer beneath offers insufficient resistance to prevent this recoil mechanism and may allow the germinal epithelium to be maintained in a retracted condition over the course of a significant period of time.

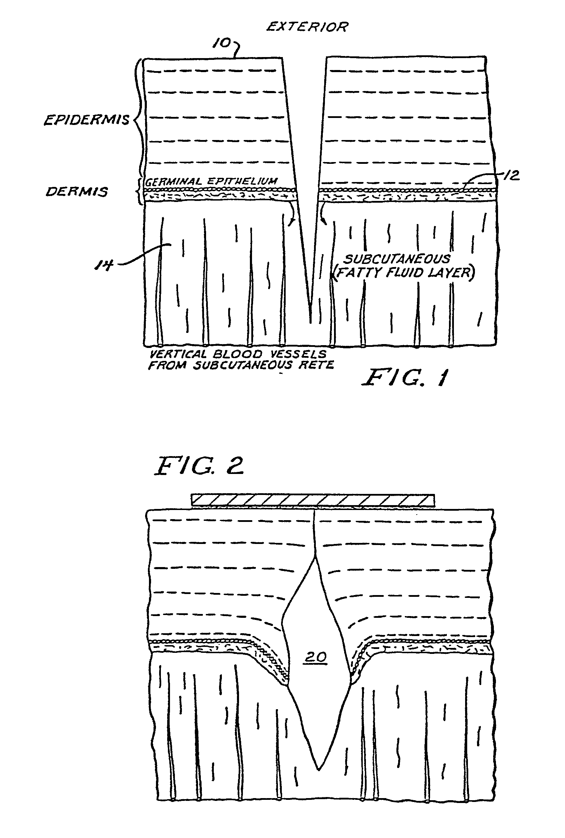

Referring specifically to Figure 1 of the drawings, it should be noted that this drawing is a schematic representation of the skin, which, as described above, is made up of many layers. For simplification, these layers can be grouped into three layers with the outer layer being called the epidermis as indicated at 10 in Figure 1. As noted above, the epidermis 10 defines the outer skin layer and forms an impermeable boundary made up of a network of dying or dead cells.

The germinal epithelium 12 defines the cell growth area where the growth of cells by multiplication and division replaces the layer above it and gradually pushes the most superficial layer towards the outside of the skin. This germinal epithelium 12 is called the dermis and is the true growth area of the skin. This, it should be noted, is one of the main skin layers involved in the healing process, since this is the layer which, when joined properly in an end-to-end relationship, will provide for optimal healing conditions and for minimal development of scar tissue.

The skin layer lying beneath the germinal epithelium or dermis 12 is the basement membrane 14 which, as noted above, is a thin layer of delicate non-cellular material of a fine filamentous texture whose principal component is collagen. The subcutaneous tissue is fatty and fluid-like and gives a cushioning effect so that the dermis 12 can slide easily over the muscles. It should be noted that this subcutaneous tissue has a poor adherent quality, or poor healing quality, since it is in a semi-fluid condition in its normal state and generally does not come together well when sutured.

Referring now to Figure 2 of the drawings, this figure shows an illustrative example of one of three common methods of skin closure of the prior art, notably, closure of a skin wound with an adhesive material. With reference to the foregoing description of the various layers of dermis, epidermis and subcutaneous tissue, it can be seen that an adhesive layer of the type shown in Figure 2 brings only the outer border of the epidermis together. In many cases this type of skin closure device does not bring the deep layer of the dermis together, in view of the fact that this device does not come into contact with the dermis in any physical manner. Accordingly, an adhesive skin closure device serves primarily as a dressing to prevent bacterial contamination but does not completely control the recoil of the dermis which can result in the creation of a space, or void. Such a void usually is filled by a haemorrhage clot or some serous clot of seepage of the normal tissue fluids into this area as seen at 20 of Figure 2. Such a clot 20 will in general eventually coagulate to define a stiffened network across which the dermis eventually will grow in an irregular manner until the dermis is complete. However, such a growth pattern of the dermis often results in an irregular underlying basement membrane and an inherent defect which will sometimes persist as an irregularity for the remainder of the life of the patient. It can readily be seen that the use of an adhesive closure, by itself, may result in significant cosmetic problems related to the formation of a permanent, undesirable scar.

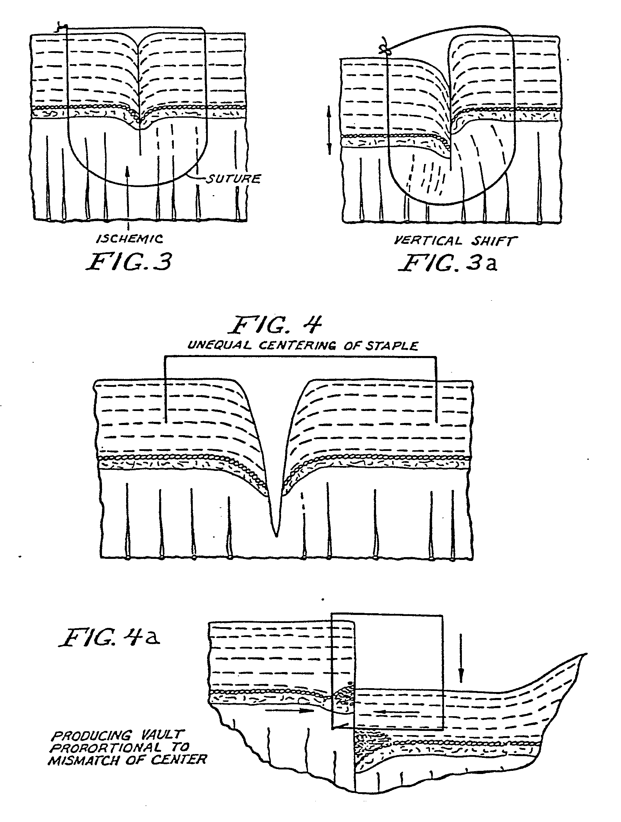

Another prior art method of wound closure, schematically represented in Figure 3, is the suture means of closure. It should be recognized that there are many forms of sutures and suture materials available for the closure of wounds. It is recognized that it is often preferable to use a non-reactive material rather than an absorbable material for sutures, since absorbable sutures will often produce a tissue reaction around the suture, giving rise to scar formation to an extent greater than that which may be cosmetically acceptable to a patient.

Suturing generally is more acceptable in certain areas, such as in the facial area, where the epidermis is of a minimum thickness. Under these circumstances, the suture line on the superficial surface of the skin tends to keep the skin aligned in generally parallel layers, thereby closing the dead space beneath the epidermis to bring the recoiled edges of the dermis into a closely aligned relationship. Such a closure generally will give an acceptable scar tissue for cosmetic purposes. However, such a closure is to some extent unpredictable and to some extent the quality of the closure depends upon the depth of the epidermis. In general, the thinner the epidermis, the greater the probability of getting the dermis properly aligned. A greater depth of tissue can make it difficult to achieve proper alignment, and consequent misalignment of the dermis can result in the development of unacceptable scar tissue. That is, a vertical shift of one layer of epidermis with respect to the other in a "step" fashion (as shown, for example, in Figure 3a), will result in misalignment of the basement membranes which can eventually result in a widened scar tissue that generally is cosmetically undesirable.

A third commonly employed prior art method for closure of a wound involves the use of staples, as in Figure 4. This closure method provides little improvement in the avoidance of scar tissue formation, and stapling can be a less predictable means for closure of a wound since every staple is individually inserted into the skin.

Generally, the skin is held everted as the staple is applied across the wound edge, and the staple, when inserted, compresses the wound with the hope that the everted edges will provide the desired alignment. This is a relatively imprecise method of obtaining the desired alignment of the basement membrane of the skin since there is little control over the membrane itself during the closure procedure. As a result, there is a significant chance of misalignment, and a resulting vertical shift can result in a less satisfactory wound closure than the simple suture. Figure 4a illustrates such a vertical shift.

Staples of the type shown in Figure 4 provide the advantage that they substantially speed the time of closure. In some instances, staples are preferable to simple suturing where the speed of surgery is an element in treatment of the patient. It should be noted, however, that unless the staple is properly placed in the centre of the wound upon closure, a vertical defect is often created which is greater when the staple is placed more off-centre with respect to the wound.

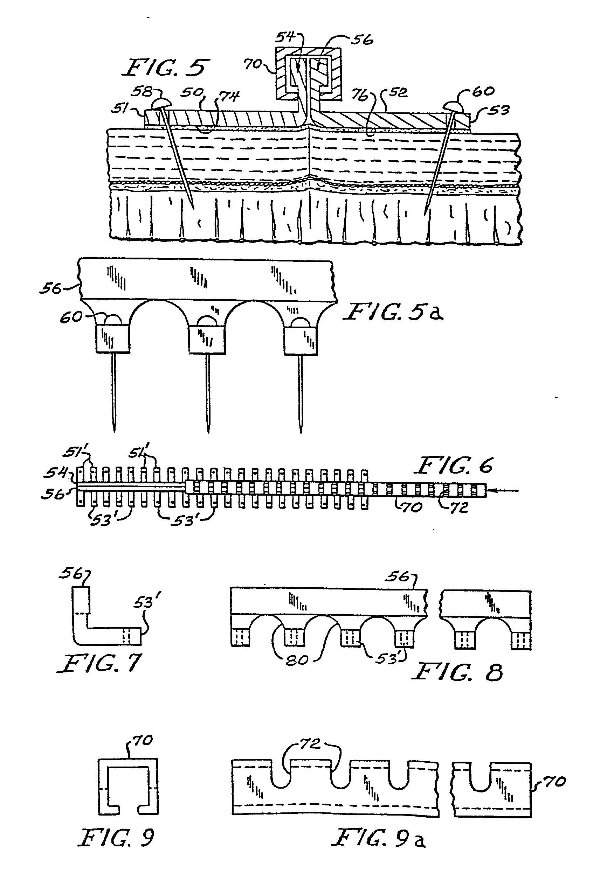

Turning now to Figures 5 to 9a, this embodiment is a skin closure device which, to a large extent, overcomes problems inherent in the use of adhesive, suturing or stapling means for skin closure, and provides a more exacting method of skin closure which minimizes the time needed to close a wound as well as the formation of scar tissue.

The device of Figures 5 to 9a includes a pair of strips 50, 52 of semi-rigid material for positioning one along either side of a wound to be closed, the strips having respective skin-contacting first portions 51, 53 which in use extend laterally away from the wound in mutually opposite directions and respective raised or upstanding second portions 54, 56 which are integral with the respective first portions 51, 53 and which in use abut against one another over the wound.

In this embodiment the skin-contacting first portion of each strip comprises a plurality of lateral fingers 51', 53' (Figure 6) spaced apart along the length of the respective strip and joined at their inner ends to the raised second portion 54, 56 of the strip.

Each of the spaced apart fingers 51', 53' of the strips 50, 52 respectively is provided with a pin 58, 60, the pins extending through the outer ends of the fingers remote from the portions 54, 56.

However, it should be noted that various wound closures may require different closure forces and it may not be necessary to include a pin at the outer terminal portion of each finger. The skin penetration portion of each pin is provided with a beveled terminal portion for the purposes to be noted hereinbelow.

The upstanding portions 54, 56 of the strips 50, 52 respectively include an enlarged portion at the upper edges thereof for cooperation with a generally U-shaped housing 70. The housing 70 has a top bridge portion and downwardly extending skirt portions on either side (Figure 9), and may be slid along the length of the strips from one end, see Figure 6, so as to engage the portions 54, 56 along their full length. When thus assembled the housing 70 holds the upstanding portions 54, 56 in abutting relation and also substantially prevents relative vertical movement between them.

An adhesive layer 74, 76 is provided along the bottom (skin-contacting) surface of the laterally extending portion 51, 53 of each strip 50 and 52. This adhesive layer is applied to the bottom surface of the fingers of the device from their outer ends to closely adjacent the edges of the wound, thereby maintaining the strips in a firmly anchored orientation with respect to the epidermis. The adhesive layer controls any lateral motion of the epidermis and it secures the device to the epidermis, thereby bringing the epidermis in a desired, edge-to-edge, closed configuration.

The laterally extending fingers 51', 53' are made sufficiently wide to spread the distribution of closure forces over a wide area, thereby reducing scar formation which otherwise might have occurred with the use of a stitch, suture or staple that might lie on the skin for a prolonged period. It generally is recognized that the healing process may take anywhere from ten to fourteen days. Sutures or staples which remain in contact with the skin during this process can give rise to skin irritation.

The pins 58 and 60 are preferably provided with a knife-like edge to allow the pins to cut the skin during insertion. This cutting action is preferable to the press fitting action characteristic of use of a conventional safety pin, or the like. The sharpened edge of the pins 58, 60 is preferably provided with an acute angle of at least 60 degrees which produces a sharp linear cut in the skin upon insertion of the pin. In this way, scar production by the pins 58, 60 is minimized. This knife-like cutting edge on the pins 58, 60 provides significant advantages.

The pins 58 and 60 may be formed such that they extend slightly inwardly toward the central part of the device as shown in Figure 5 to provide enhanced wound closure forces. This inward pitch may preferably be on the order of about 15 degrees.

The pins are preferably formed of a high-grade stainless surgical steel which is non-reactive to the body and which will produce no significant soft tissue reaction around it which might be detrimental in the production of scar tissue.

The laterally extending portions 51 and 53, which in the embodiment of Figures 5 to 9a are defined by spaced apart fingers 51' and 53' are integrally attached to the upstanding portions 54 and 56, respectively. The skin-contacting surface of each finger is joined by a smooth upwardly curved surface to the surface of the upstanding portion integral therewith and which abuts the corresponding surface of the other strip. The two surfaces define a gap immediately above the wound to allow the skin to be everted during the healing process. This eversion of the skin is highly controlled by the shape of the curve joining the laterally extending and upstanding portions of the strips 50 and 52 and permits a slight movement of the inner portion of the strips in relation to the wound being controlled by the device. This action in cooperation with the action of the pins 58, 60 controls, via the vertex of the wound, the deeper dermis and tends to neutralize the inversion effect or recoil of the basement membrane during the healing process. The result is that a straight epithelial membrane is defined in end-to-end relationship which often will produce almost no void or vertical step in the depth of the wound.

The closure housing 70, when slid in place, locks the two strips 50 and 52 in assembled relation and prevents overriding or vertical motion of one side of the wound with respect to other, thereby maintaining proper alignment of the closure. The closure housing 70 may be serrated as shown in Figure 9a of the drawings with the serrations being defined by apertures 72 spaced along the bridge portion of the housing to provide for flexibility of the assembled apparatus and to allow for free drainage from between the upstanding portions 54 and 56 strips. In use, the closure housing 70 is slid in place over the upstanding portions 54 and 56 to provide rapid approximation of each skin edge being controlled by the device, whether the device is applied after the wound was created or prior to incision of the wound.

The pins 58, 60 are individually pushed into the skin through the pre-formed openings of the fingers of the strips. A multiple pin insertion apparatus can be provided for this purpose which requires a minimum amount of force to be exerted at the skin edge upon insertion of the pins. It should be noted that, in the embodiment of Figures 5 to 9a, the pins have not been incorporated or molded into the strips. This allows the surgeon to remove the pins individually or in groups in accordance with defined surgical procedures. The pins are provided with an enlarged safety head to prevent migration of the pin into the depth of the wound and also to allow for easy removal of the pin with a simple forceps action, thereby leaving the strips adherent to the skin to maintain the wound in essentially controlled manner once the serous clot and early epithelization has occurred but before full strength has been obtained.

It should be noted that in the embodiment of Figures 5 to 9a suitable drainage sites are provided by spaces 80 (Figure 8) at the junction of the fingers and the portions 54 and 56, such spaces being a continuation of the lateral spaces between the fingers partially upwardly into the respective raised or upstanding portions 54 and 56. These drainage sites 80 allow for free drainage to occur from the wound and for the removal laterally of the wound of any excess serous secretion that may build up at the wound edges or to accommodate any hematoma that may build up and tend to lift the apparatus which would subsequently result in discharge of material from the drainage sites 80.

It also should be noted that the drainage sites allow for easy examination of the wound edges to insure that the wound has been carefully brought in apposition and has not been left excessively everted or distracted by the application of the apparatus.

It should be noted that blood is supplied to the skin via a reticular network which is a vertically oriented system. The device of Figures 5 to 9a, unlike sutures or the like, does not interfere or put under compression the skin edges and allows for normal post-operative swelling to occur and recede without interfering with the blood supply to the vital healing tissue. This is an important aspect of the embodiment of Figures 5 to 9a and is a characteristic of the design and use of this device in assembled relation in holding the wound in a closed orientation during the healing process.

It can readily be seen that the strips 50 and 52 and the closure housing 70 may be of any desired length and are made of material which may readily be cut with any convenient apparatus to the exact length desired by the physician or surgeon.

In one preferred embodiment of the device of Figures 5 to 9a, the strips and closure housing are made of nylon which in its characteristic form will have sufficient flexibility in use, so that the apparatus may be placed in a lateral spaced apart configuration with respect to the wound in any desired configuration and made to adapt the characteristic nature of the wound. This flexibility characteristic is, of course, enhanced in the preferred embodiment of Figures 5 to 9a by the use of the spaced apart fingers as the skin-contacting portions 51 and 53 and the drainage sites 80 associated therewith. The removal of this material, of course, permits increased flexibility of the apparatus for convenience in use. It should be noted, however, that a material which is too flexible may defeat the purpose of the apparatus if the material is not sufficiently rigid to maintain the wound closure in proper orientation. Therefore, it is important to provide the device with a proper degree of flexibility which permits a preferred orientation of the device with respect to the wound while still being sufficiently rigid to hold the wound in the desired closed orientation during the healing process.

During the application procedure it should be noted that the pins are in a fully retracted position and do not extend substantially below the lower face of the laterally extending fingers 51' and 53'. This permits initial application in the non-penetrating area of the skin without the need for holding the skin edges with a forceps and further traumatizing the delicate skin edges. It should be noted that the undersides of the laterally extending fingers 51' and 53' are provided with the adhesive interface which will orient the strips 50, 52 with respect to the skin. Accordingly, the device can be put on atraumatically and closed without any forceps touching the skin edge. Further, the adhesive layer on the underside of the laterally extending fingers is such that if the device is applied in an unsatisfactory orientation it can easily be removed and reapplied in the proper orientation of the strips 50 and 52 with respect to the wound. The pins may be inserted individually or in groups to provide a rapid rate of closure which is substantially in excess of commonly used suturing techniques and which may even be faster than the application of staples for wound closure. Since there is substantially no motion between the pins which transfix the skin and the surrounding skin (a feature provided by the adhesive layer associated with the strips), there is substantially no motion of the skin at the site of the pins. The adhesive layer closes the skin puncture at the pin to bacterial contamination over the course of the healing process and also reduces the motion of the pins which could result in the production of scar tissue. It has been found that this combination of pins and adhesive along with the sharp pin knife edge produces an atraumatic and almost scarless pin site.

The device of Figures 5 to 9a can be applied either after the wound has been created at the time of surgery or before the skin is cut and then subsequently separated at the time of the surgical incision. This latter approach minimizes the time required to close the wound. It also should be noted that the removal of the device from the skin can occur in two ways: the pins can be removed one at a time at the time of healing, or alternatively one edge of the device can be lifted and peeled off. There is little pain involved in this manoeuvre since the pins are typically in place for a period of more than forty-eight hours, and removal of the pins in such a manner will typically not cause patient discomfort. Patient discomfort in the removal of sutures or staples can be greater since staples tend to catch the patient on the deep elbow as they are removed.

It should be noted that an important advantage of the skin closure device of Figures 5 to 9a is that it allows the basement membrane of the skin to be aligned properly. This is a significant factor in the reduction of the production of scar tissue and results in a more cosmetically acceptable wound closure.

The speed of a wound closure is an important factor, since in many instances decreased operating time is highly desirable to reduce the anesthetic risk and to reduce the incidence of bacterial contamination which might occur in a prolonged operation. The improved closure device of Figures 5 to 9a provides the further important advantage of extremely rapid skin closure.

Another desirable feature of the embodiment of Figures 5 to 9a is its versatility in that this embodiment allows the transfixing pins to be removed individually at the surgeon's discretion without the removal of the entire apparatus. This approach leaves the device intact while still allowing for the early removal of pins as may be appropriate and indicated in certain rapidly healing areas such as the face, head and neck.

The pins utilized in the embodiment of Figures 5 to 9a may be formed of stainless steel, chrome, cobalt or titanium alloys, or some other suitable non-reactive rigid material.

The ease of application of the device with respect to the wound permits its use in a wide range of elective surgical procedures without the need to change traditional methods of skin and wound preparation.

For example, as mentioned above, when used in a surgical procedure, the closure device of Figures 5 to 9a may first be placed so that the raised abutting portions of the strips extend along the area to be incised. The closure housing is then removed from the device and the incision then may be made in the region defined between the abutting portions. It can readily be seen that accurate re-alignment of the incised tissue can quickly be realized by bringing the strips back together in an abutting relation and placing the closure housing over the upstanding portions of the strips to hold the latter in assembled relation as schematically represented in Figure 5 of the drawings.

To obtain proper wound healing, it is important to bring the epidermis, the dermis, as well as the outer layers of the subcutaneous tissue into close apposition and to maintain this orientation during the healing process. This maintenance of orientation is easily obtained with the device of Figures 5 to 9a, and a skin wound can be closed extremely rapidly.

Preferably, all of the materials employed in the device of Figures 5 to 9a are non-reactive with respect to human tissue and, subsequently, minimum scar formation will result from the use of this device.

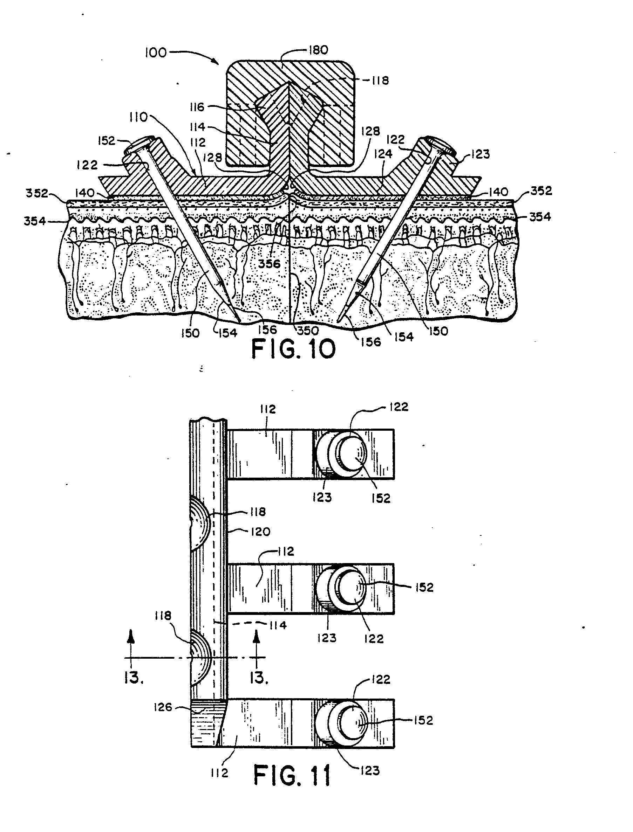

Turning now to Figures 10 to 19, a second embodiment of this invention is shown which is similar to the first embodiment described above. In Figure 10 the reference numeral 350 has been used to designate a skin wound, the reference numeral 352 has been used to designate superficial skin layers (the epidermis), the reference numeral 354 has been used to designate the dermis, and reference numeral 356 has been used to designate the marginal edge of the skin wound.

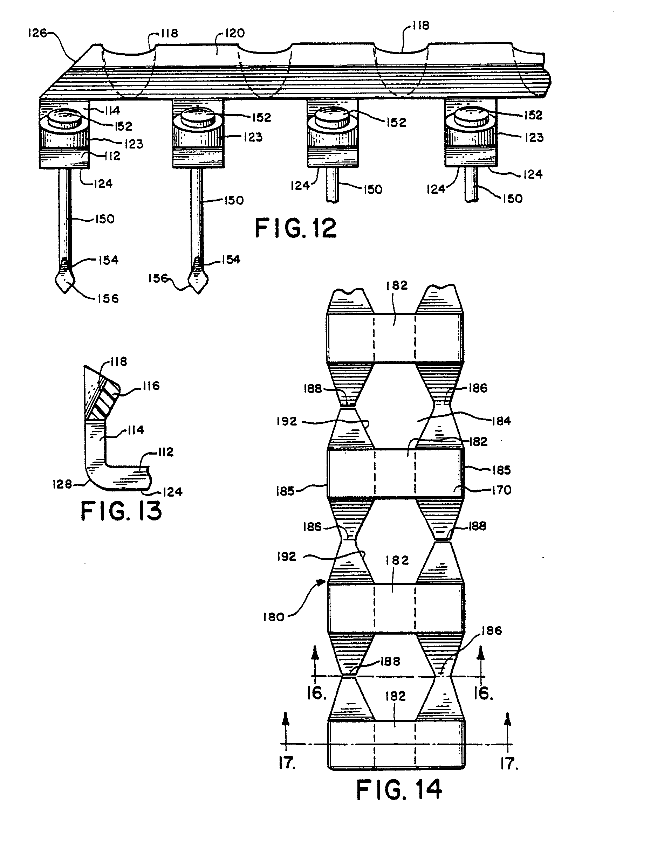

This second embodiment 100 includes two pin harnesses 110. The relationship between these two pin harnesses 110 in the assembled configuration is shown in Figures 10. Figures 11 through 13 show various additional views of one of the pin harnesses 110 in order further to define its structure. As shown in these figures, each of the pin harnesses 110 includes an array of skin contacting members 112. Each of these skin contacting members 112 is shaped as an elongated, finger-like projection which is mounted to a respective upright member 114. Each of the upright members is in turn mounted to a longitudinal rail 116 which extends across the plurality of the skin contacting members 112. The members 112, 114 and the rail 116 cooperate to form a plurality of apertures 120 therebetween. As best shown in Figure 12, the rail 116 is maintained above the level of the skin, and the apertures 120 allow free drainage of fluids from the skin wound. In addition, the rail 116 defines a spaced plurality of recesses 118 which serve further to enhance drainage from the skin wound. As best shown in Figures 12, each end 126 of each of the rails 116 is preferably bevelled to facilitate assembly of the skin closure device. Each of the skin contacting members 112 defines a respective pin guide 112 which terminates at its upper end in a boss 123. As best shown in Figure 10, each of the skin contacting members 112 defines a respective skin contacting surface 124 along its underside surface. The innermost surface of each of the skin contacting members 112 is provided with a radius of curvature 128 to allow for skin eversion adjacent the skin wound.

In this preferred embodiment, the pin guides 112 are spaced such that two pin guides 122 are provided per centimetre on each pin harness 110 (five pin guides per inch). A preferred range for the pin guide spacing is between 1.2 and 3.2 pin guides 112 per centimetre (between 3 and 8 pin guides per inch). Simply by way of illustration and not by way of limitation, each of the radiused portions 128 is in this preferred embodiment provided with a radius of curvature of 0.035 inches. The pin guides 122 are sized to receive the respective pins in a sliding fit so as to orient the pins properly for penetration of the skin. The angle of the pin guides with respect to the perpendicular to the skin surface can vary within the range of about 0 degrees to about 35 degrees. Within this range, the preferred angle of the pin guides 122 with respect to the perpendicular is about 15 degrees to about 30 degrees. In the presently preferred embodiment, a pin guide angle of about 20 degrees with respect to the perpendicular is considered optimum.

Each of the skin contacting surfaces 124 of the pin harnesses 110 is provided with an adhesive layer 140. This adhesive layer 140 extends around the lower portion of the pin guides 122 as well as to a point closely adjacent the radiused portions 128. Preferably, this adhesive layer is precoated onto the skin contacting surfaces 124 during manufacture.

A wide variety of skin adhesives are suitable for use with the present invention. However, in the presently preferred embodiment the adhesive layer 140 is formed of a skin adhesive distributed by Dow Corning as Adhesive No. 355. This adhesive is a solution of 18.5 percent by weight of dimethylpolysiloxane in trichlorotrifluorethane (Freon (trade mark)). Of course, this example of a preferred adhesive is provided merely by way of illustration and not of limitation.

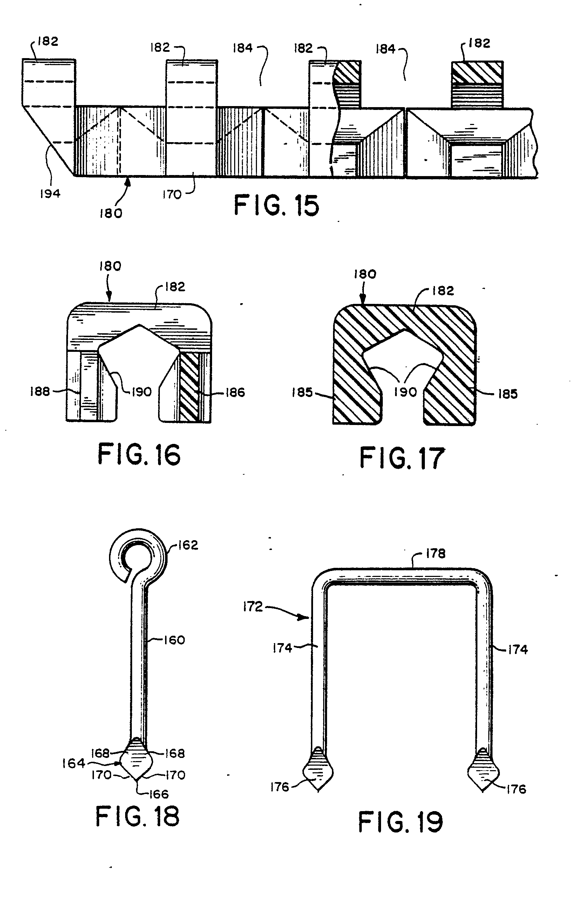

As shown in Figure 10, the skin closure device 100 also includes two sets of pins 150. Each of these pins 150 is provided with an enlarged head 152 as well as with a point 154 which defines a knife-like cutting edge 156. The nature of the points 154 is best shown in Figures 18 and 19, which disclose two alternate embodiments of the pin 150. In Figure 18, the pin 160 is provided with a looped end section 162 and a point 164 which defines an apex 166. In addition, the point 164 defines two enlarged shoulders 168, and a knife-like cutting edge 170 which extends along the sides of the shoulders 168 to the apex 166.

The pin shown in Figure 19 is yet another alternative embodiment 172 which includes two pin shafts 174, each of which defines a respective point 176 of the type described above. A pin shank 178 connects the two shafts 174 together such that the two shafts 174 form a unit. This embodiment 172 provides the advantage of rapid pin insertion and removal. Simple finger pressure is sufficient to insert the pin 172 easily due to the area and spacing of the shank 178. In each of the pins 150, 160, 172, the angle defined by the cutting edges 156, 170 is preferably about 60 degrees.

By way of example only, the pins can be made of type 316 stainless steel. Of course, other metals or plastics materials of suitable mechanical properties can be substituted. Merely by way of example, in the preferred embodiments discussed above, the shaft of each of the pins is about 0.0533 cm (0.021 inches) in diameter, and the diameter of the pin guides is about 0.0538 cm (0.0212 inches) in diameter. These dimensions provide a close fit of the pins in the pin guides in order to orient the pins properly. In each case, both the heads 152, 162, 178 and the points 154, 164, 176 of the pins are enlarged so as to prevent the pins from escaping from the pin guides 122. In this way, each pin is slidably captured within its pin guide and cannot readily be lost or removed from the pin harness. In this preferred embodiment, the presently preferred length of the pin 150 from the tip of the point 154 to the underside of the head 152 is about 0.8 cm (0.311 inches). An alternative embodiment provides a pin 150 with a length of about 0.58 cm (0.230 inches).

Turning now to Figures 14 through 16, the skin closure device 100 also includes a housing 180. This housing 180 is an elongated member which defines a U-shaped cross section. The housing 180 is made up of a plurality of bridge sections 182 which interconnect two opposed side skirts or sidewalls 185. An array of cross slots 184 are positioned between the bridge portions 182 to intersect the channel 190 defined between the sidewalls 185. These cross slots 184 provide openings or apertures 192 which facilitate the drainage of fluids from the skin wound. As best shown in Figure 14, each of the sidewalls 185 defines alternatively positioned narrowed regions 186 and severed regions 188. When the housing 180 is bent laterally in the plane of Figure 14, the severed regions 188 open and the narrowed regions 186 bend to allow the entire housing 180 to bend laterally. Preferably, the housing 180 is substantially more rigid to bending in a plane perpendicular to the plane of Figure 14 than in the plane of Figure 14. Preferably, both ends 194 of the housing 180 are bevelled to facilitate assembly of the skin closure device.

In the preferred embodiment 100 of Figures 10 to 19, the pin harnesses 110 and the housing 180 are preferably formed of a high density ethylene hexene-1 copolymer such as the material marketed by Phillips Chemical Company, Battlesfield, Oklahoma as Marlex type HHM 5502 (Marlex is a trade mark). This material has been found to provide excellent properties in terms of minimum skin reaction and optimum rigidity. The flexibility of this material is temperature dependent, and it has been found to be suitably rigid when the skin closure device 100 is being installed adjacent the skin wound and yet to conform properly to the contour of the skin wound as the skin closure device warms to skin temperature. Preferably, the pin harnesses 110 and the housing 180 are injection-molded and the pin guides 122 are cored. It should be understood that it is not essential in all preferred embodiments that the pin guides 122 be circular in cross section; square pin guides or pin guides of other cross-sectional shape can be substituted. The severed regions 188 in the housing 180 can be formed either by injection molding or by a cutting operation subsequent to injection molding.

The pins 150 of the preferred embodiment described above are preferably first formed with a cold forged head and a standard conical point, and are then assembled in the respective pin guides 122. The point 154 which defines the cutting edges 156 is then formed in a three-step cold forging operation. The first step is a cutting step to trim off the pin at the desired length. This cutting operation is performed in a first station of a suitable metal forming machine. The second step is to coin the points at a second station of the metal forming machine to a paddle shape having a thickness in the range of about 0.005 cm (0.002 inches) to about 0.018 cm (0.007 inches). Then, the point 154 is cut in a third station to a suitable diamond shape. This cutting operation creates the cutting edges 156. By the process described above, the points 154 can be formed in the pins 150 only after the pins 150 have been inserted in the pin guides 122. In this way, the pins 150 are captured securely within the pin guides 122. Preferably, the stainless steel from which the pins 150 are formed is work hardened to a tensile strength of between 1.448 and 1.586 GPa.

Preferably, the skin closure device 100 (including the pin harnesses 110, the adhesive layer 140, the pins 150, and the housing 180) is packaged in a suitable material along with a pin-pushing device formed of Marlex (trade mark) as described above and defining a recessed end sized to fit over the heads 152 of the pins 150. Of course, other types of pin pushing devices, such as forceps-like devices adapted to insert a number of pins at once, can also be used.

These packages also preferably include a quantity of a suitable adhesive. In the preferred embodiment, this adhesive is contained in a glass bottle having a permeable neoprene applicator. In the presently preferred embodiment, an adhesive of the type described above is used. However, in order to provide reduced drying times, the Type 355 Dow Corning adhesive is allowed partially to evaporate before the glass bottle is sealed. In the presently preferred embodiment, 11 cc of Type 355 adhesive is allowed to evaporate down to about 6 cc before the bottle is sealed.

Once the package has been assembled, it is then preferably sterilized. This can be done by placing a large number of the packages near a Cobalt 60 source of gamma radiation. Preferably, the packages are rotated within a radiation chamber until a dosage of about 2.5 Mrads is obtained. In this way, the entire skin closure device along with its adhesive is completely sterilized.

The skin closure device 100 described above in conjunction with Figures 10 through 19 can be used in the manner of the preferred embodiment of Figures 5 to 9a. Preferably, adhesive from the applicator is applied to the skin on both sides of the skin wound. Then the skin contacting surfaces 124 of the pin harness 110 are adhesively bonded to the skin in the desired position and the pins 150 are then pushed into the skin. Preferably, the epidermis is adhesively secured to the pin harnesses at points closely adjacent to the marginal edge of the skin wound. In order to obtain optimum results, this region of adhesive bonding should extend to within 0.5 millimeters of the skin wound.

As described above, the skin closure device 100 can either be applied to unbroken skin prior to the formation of a surgical incision, or alternatively it can be applied to the marginal edges of the skin wound after the skin wound has been formed. In either case, the housing 180 is used to approximate the rails 116 of the pin harnesses 110 in order to close the skin wound quickly, efficiently and precisely. Generally, no occlusive dressing is needed.

The preferred embodiment 100 described above provides a number of important advantages. Since the skin contacting surfaces 124 are bonded by the adhesive layer 140 to the epithelium at points closely adjacent to the marginal edge of the skin wound, the skin contacting members 112 serve to bring together and align the epithelium layers precisely. Furthermore, the pins 150 are dimensioned such that the points 154 mechanically engage the dermis. The broad surface of the points 154 serve to enhance this mechanical engagement. When the housing 180 is used bring the points 154 of the pins 150 together, the pins 150 serve to mechanically engage the dermis and to cause the marginal edges of the dermis adjacent the wound to come together in proper alignment. In this way, the skin closure device 100 serves automatically and reliably to align both the epithelium layer and the dermis in precise, edge-to-edge contact and alignment. In this way, healing of the skin wound is facilitated and the formation of scar tissue at the skin wound is minimized.

Furthermore, the embodiment of Figures 10 through 19 provides pins 150 which are slidable with respect to the pin harnesses 110, yet which are securely captured in the pin guides 122. This structure allows individual pins 150 to be removed from the skin if desired, yet substantially prevents the loss of individual pins. If desired, recesses can be formed in the underside of the pin guides 122 to receive the pin points 154.

Yet another advantage of this embodiment is that the adhesive layer 140 extends adjacent to and around the pins 150. The adhesive layer 140 operates to immobilize the epithelium layer of the skin around the pins 150 in order to minimise relative motion therebetween. Such relative motion can result in the formation of undesirable scar tissue. The layer of adhesive 140 around the pins 150 further serves to seal the opening in the epithelium layer made by the pins 150 in order to reduce infection and the introduction of foreign material into the epithelium.

## Claims

1. A device for use in the rapid closure of skin wounds, comprising a pair of strips of material (50, 52) for positioning one on either side of a wound to be closed, the strips having respective skin-contacting first portions (51, 53) which in use extend laterally away from the wound in mutually opposite directions, the strips further being of semi-rigid material and having respective raised second portions (54, 56) which are integral with the respective first portions (51, 53) and which in use abut against one another over the wound, securing means (70) adapted to engage and hold together in abutting relationship the raised second portions (54, 56) of the strips and to limit vertical movement therebetween, and a layer of adhesive (74, 76) on the skin-contacting surface of the first portion (51, 53) of each strip for adherence of the strips to the skin, the layer of adhesive on the first portion of each strip extending to adjacent the edges of the wound, characterised by:
a plurality of pins (58, 60) disposed along the length of each strip (50, 52) for penetrating the skin, the pins being located along the length of the first portion (51, 53) of each strip and being laterally spaced from the respective second portion (54, 56) of the strip, each pin (58, 60) further being slidable relative to the first portion (51, 53) so as to permit the pin to be pushed downwardly into the skin from an initial position wherein the pin does not penetrate the skin.

2. A skin closure device according to claim 1, further including a plurality of apertures (80) along the length of each strip at least in the region of the junction of the first (51, 53) and second (54, 56) portions to provide wound drainage sites.

3. A skin closure device according to claim 1 or 2, wherein the skin-contacting first portion (51, 53) of each strip comprises a plurality of lateral fingers (51', 53') spaced apart along the length of the strip and joined at their inner ends to the raised second portions (54, 56) of the strip.

4. A skin closure device according to claim 3 when dependent on claim 2, wherein in each strip the spaces between the fingers (51', 53') extend partially upwardly into the respective raised second portion (54, 56) to provide the said drainage sites.

5. A skin closure device according to claim 3 or 4, wherein in each strip the pins (58, 60) are located adjacent the ends of the fingers (51', 53') remote from the respective raised second portion (54, 56).

6. A skin closure device according to any preceding claim, wherein the abutting raised second portions (54, 56) are spaced apart at their lower regions to define a gap immediately above the wound to permit eversion of the skin during healing.

7. A skin closure device according to claim 6, wherein in each strip the skin-contacting surface of the first portion (51, 53) is joined by a smooth upwardly curved surface to the surface of the second portion (54, 56) which abuts the corresponding surface of the other strip, the two curved surfaces defining the said gap.

8. A skin closure device according to any preceding claim, wherein the pins (58, 60) have enlarged heads to prevent their movement fully through the first portion (51, 53) of the respective strip.

9. A skin closure device according to any preceding claim, wherein the pins (58, 60) have a knife-like tapered portion at the end thereof to be inserted into the skin.

10. A skin closure device according to any preceding claim, wherein the securing means comprises a housing (70) slidable along the length of the strips from one end to engage the raised second portions (54, 56).

11. A skin closure device according to claim 10, wherein the housing (70) is of generally U-shaped cross-section with a top bridge portion and downwardly extending skirt portions on either side which in use embrace the raised abutting second portions (54, 56) of the strips.

12. A skin closure device according to claim 11, wherein the bridge portion of the housing is apertured (72) along its length.

13. A skin closure device according to any preceding claim, wherein the layer of adhesive (74, 76) also extends to closely adjacent the pin locations.

## Revendications

1. Dispositif destiné à être utilisé pour la fermeture rapide des plaies de la peau, comprenant deux bandes de matière (50, 52) destinées à être positionnées de part et d'autre de la plaie à fermer, lesdites bandes comportant chacune une première partie (51, 53) au contact de la peau, qui, lors de leur utilisation, s'étendent latéralement en s'éloignant de la plaie dans des directions opposées l'une par rapport à l'autre, les bandes étant en outre en matériau semi-rigide et présentant chacune des secondes parties (52, 56) surélevées qui font partie de la même pièce que lesdites premières parties (51, 53) respectivement, et qui, lors de leur utilisation reposent l'une contre l'autre au-dessus de la plaie; des moyens de fixation (70) permettant de maintenir en contact l'une avec l'autre lesdites secondes parties surélevées (54, 56) des bandes et de limiter leurs mouvements verticaux relatifs; ainsi qu'une couche de matière adhésive (74, 76) sur la surface en contact de la peau de ladite première partie (51, 53) de chacune des bandes pour permettre l'adhérence des bandes à la peau, la couche de matière adhésive sur ladite première partie de chaque bande s'étendant jusqu'à proximité des bords de la plaie, caractérisé par:

— une pluralité d'épingles (58, 60) disposées sur la longueur de chaque bande (50, 52) pour pénétrer dans la peau, les épingles étant situées sur la longueur de la première partie (51, 53) de chaque bande et étant espacées latéralement de ladite seconde partie (54, 56) respectivement de la bande, chaque épindle (58, 60) pouvant en outre glisser par rapport à ladite première partie (51, 53) de façon à permettre de repousser l'épingle vers le bas et à l'intérieur de la peau en partant de la position initiale dans laquelle l'épindle ne pénètre pas dans la peau.

2. Dispositif pour refermer la peau selon la revendication 1, comprenant en outre une pluralité d'ouvertures (80) sur la longueur de chaque bande, au moins dans la zone de jonction de la première (51, 53) et de la seconde (54, 56) parties, pour réaliser des sites de drainage de la plaie.

3. Dispositif pour refermer la peau selon la revendication 1 ou 2, dans lequel la première partie en contact de la peau (51, 53) de chaque bande comprend une pluralité de doigts latéraux (51', 53') espacés les uns des autres sur la longueur de la bande et reliés par leurs extrémités inférieures auxdites secondes parties surélevées (54, 56) de la bande.

4. Dispositif pour refermer la peau, selon la revendication 3, lorsqu'elle est dépendante de la revendication 2, dans lequel, pour chaque bande, les espaces séparant les doigts (51', 53') s'étendent en partie vers le haut à l'intérieur des secondes parties surélevées (54, 56) respectivement, pour constituer lesdits sites de drainage.

5. Dispositif pour refermer la peau, selon la revendication 3 ou 4 dans lequel, pour chaque bande, les épingles (58, 60) sont situées à proximité des extrémités des doigts (51', 53') les plus éloignées desdites secondes parties surélevées (54, 56) respectivement.

6. Dispositif pour refermer la peau selon l'une quelconque des revendications précédentes, dans lequel lesdites secondes parties surélevées (54, 56) en contact l'une de l'autre sont espacées l'une de l'autre dans leur zone intérieure pour définir un passage immédiatement au-dessus de la plaie permettant l'éversion de la peau lors de la cicatrisation.

7. Dispositif pour refermer la peau, selon la revendication 6, dans lequel, pour chaque bande, la surface au contact de la peau de la première partie (51, 53) est reliée, par une surface légèrement incurvée vers le haut, à la surface de la seconde partie (54, 56) qui est en contact de la surface correspondante de l'autre bande, les deux surfaces incurvées définissant ledit passage.

8. Dispositif pour refermer la peau selon l'une quelconque des revendications précédentes, dans lequel les épingles (58, 60) présentent des têtes élargies pour éviter qu'elles traversent complètement la première partie (51, 53) de la bande correspondante.

9. Dispositif pour refermer la peau, selon l'une quelconque des revendications précédentes, dans lequel les épingles (58, 60) présentent une partie conique en lame de couteau, à leur extrémité destinée à être introduite à l'intérieur de la peau.

10. Dispositif pour refermer la peau, selon l'une des revendications précédentes, dans lequel les moyens de fixation comprennent un boîtier (70) pouvant glisser sur la longueur des bandes à partir de l'une des extrémités, pour maintenir en contact les secondes parties (54, 56) surélevées.

11. Dispositif pour refermer la peau, selon la revendication 10, dans lequel le boîtier (70) est de section transversale approximativement en forme de U, avec une partie supérieure de fond, et des flancs s'étendant vers le bas de chaque côté du fond pour entourer lors de leur utilisation les secondesparties surélevées (54, 56) des bandes en contact l'une avec l'autre.

12. Dispositif pour refermer la peau selon la revendication 11, dans lequel la portion de fond du boîtier est ajourée (72) sur sa longueur.

13. Dispositif pour refermer la peau selon l'une quelconque des revendications précédentes, dans lequel la couche d'adhésif (74, 76) s'étend également jusqu'à proximité immédiate des épingles.

**Patentansprüche**

1. Vorrichtung zur Verwendung beim Schnellverschluß von Hautwunden mit einem Paar von Materialstreifen (50, 52), von denen einer zu jeder Seite einer zu schließenden Wunde angeordnet ist, wobei die Streifen jeweilige die Haut berührende erste Teile (51, 53) aufweisen, die im Gebrauch seitlich von der Wunde fort in Richtungen entgegengesetzt zueinander sich erstrecken, wobei die Streifen weiter aus halbsteifem Material bestehen und jeweilige erhabene zweite Teile (54, 56) haben, die einstückig mit den jeweiligen ersten Teilen (51, 53) sind und die im Gebrauch gegeneinander über der Wunde anliegen, Festhalteeinrichtungen (70), die so ausgebildet sind, daß sie aneinander anliegend die hochstehenden zweiten Teile (54, 56) der Streifen erfassen und zusammenhalten und die Vertikalbewegung hierzwischen begrenzen, und mit einer Lage Klebstoff (75, 76) auf der die Haut berührenden Oberfläche des ersten Teils (51, 53) jedes Streifens, um die Streifen an der Haut zum Haften zu bringen, wobei die Klebstoffschicht auf dem ersten Teil jedes Streifens sich bis in die Nachbarschaft der Ränder der Wunde erstreckt, gekennzeichnet durch eine Vielzahl von Stiften (58, 60), die längs der Länge jedes Streifens (50, 52) zum Eindrigen in die Haut angeordnet sind, wobei die Stifte über die Länge des ersten Teils (51, 53) jedes Streifens angeordnet und unter seitlichem Abstand zum jeweiligen zweiten Teil (54, 56) des Streifens angeordnet sind und jeder Stift (58, 60) weiterhin relativ zum ersten Teil (51, 53) verschiebbar ist, so daß der Stift nach unten in die Haut aus der Ausgangslage, in der der Stift in die Haut nicht eindringen kann, geschoben werden kann.

2. Hautverschlußvorrichtung nach Anspruch 1, weiterhin mit einer Vielzahl von Öffnungen (80) über die Länge jedes Steifens, wenigstens im Bereich des Übergangs der ersten (51, 53) und zweiten (54, 56) Teile zur Schaffung von Wunddrainageorten.

3. Hautverschlußvorrichtung nach Anspruch 1 oder 2, dadurch gekennzeichnet, daß der die Haut kontaktierende erste Teil (51, 53) jedes Streifens eine Vielzahl seitlicher Finger (51', 53') aufweist, die unter Abstand entlang der Länge des Streifens angeordnet und an ihren innen gelegenen Enden mit den hochstehenden zweiten Teilen (54, 56) des Streifens verbunden sind.

4. Hautverschlußvorrichtung nach Anspruch 3, wobei dieser abhängig von Anspruch 2 ist, dadurch gekennzeichnet, daß in jedem Streifen die Räume zwischen den Fingern (51', 53') teilweise nach oben in den jeweiligen angehobenen zweiten Teil (54, 56) unter Bildung dieser Drainageorte reichen.

5. Hautverschlußvorrichtung nach Anspruch 3 oder 4, dadurch gekennzeichnet, daß in jedem Streifen die Stifte (58, 60) benachbart den Enden der Finger (51', 53') angeordnet sind, die entfernt von dem jeweiligen angehobenen zweiten Teil (54, 56) liegen.

6. Hautverschlußvorrichtung nach einem der vorhergehenden Ansprüche, dadurch gekennzeichnet, daß die aneinander anstoßenden hochstehenden zweiten Teile (54, 56) an ihren unteren Bereichen unter Abstand voneinander vorgesehen sind, um einen Spalt unmittelbar oberhalb der Wunde zu bilden und während des Heilens die Eversion der Haut zu ermöglichen.

7. Hautverschlußvorrichtung nach Anspruch 6, dadurch gekennzeichnet, daß in jedem Streifen die Haut berührende Oberfläche des ersten Teils (51, 53) über eine glatte nach oben gekrümmte Fläche mit der Oberfläche des zweiten Teils (54, 56) verbunden ist, welche der entsprechenden Fläche des anderen Streifens anliegt, wobei die beiden gekrümmten Flächen diesen Spalt definieren.

8. Hautverschlußvorrichtung nach einem der vorhergehenden Ansprüche, dadurch gekennzeichnet, daß die Stifte (58, 60) vergrößerte Köpfe haben, um deren volle Bewegung durch den ersten Teil (51, 53) des jeweiligen Streifens zu verhindern.

9. Hautverschlußvorrichtung nach einem der vorhergehenden Ansprüche, dadurch gekennzeichnet, daß die Stifte (58, 60) einen messerartig sich verjüngenden Teil an ihrem Ende zum Einführen in die Haut haben.

10. Hautverschlußvorrichtung nach einem der vorhergehenden Ansprüche, dadurch gekennzeichnet, daß die Festhalteeinrichtung ein Gehäuse (70) umfaßt, das entlang der Länge der Streifen von einem Ende gleitfähig ausgebildet ist, um die hochstehenden zweiten Enden (54, 56) zu erfassen.

11. Hautverschlußvorrichtung nach Anspruch 10, dadurch gekennzeichnet, daß das Gehäuse (70) von im allgemeinen U-förmigem Querschnitt mit einem Kopfbrückenteil und nach unten reichenden Schürzenteilen zu beiden Seiten sind, die im Betrieb die hochstehenden aneinander anliegenden zweiten Teile (54, 56) der Streifen umfassen.

12. Hautverschlußvorrichtung nach Anspruch 11, dadurch gekennzeichnet, daß der Brückenteil des Gehäuses entlang seiner Länge mit Öffnungen (72) versehen ist.

13. Hautverschlußvorrichtung nach einem der vorhergehenden Ansprüche, dadurch gekennzeichnet, daß die Klebstoffschicht (74, 76) auch bis nahe benachbart den Orten der Stifte reicht.

EXTERIOR

10

EPIDERMIS

GERMINAL EPITHELIUM

DERMIS

12

14

SUBCUTANEOUS
(FATTY FLUID LAYER)

VERTICAL BLOOD VESSELS
FROM SUBCUTANEOUS RETE

*FIG. 1*

*FIG. 2*

20

1

ISCHEMIC

*FIG.3*

VERTICAL SHIFT

*FIG.3a*

SUTURE

*FIG. 4*

UNEQUAL CENTERING OF STAPLE

*FIG. 4a*

PRODUCING VAULT
PROPORTIONAL TO
MISMATCH OF CENTER

FIG. 5

FIG. 5a

FIG. 6

FIG. 7

FIG. 8

FIG. 9

FIG. 9a

FIG. 10

FIG. 11

0 092 334

FIG.12

FIG.13

FIG.14

5

FIG. 15

FIG. 16

FIG. 17

FIG. 18

FIG. 19